# EUROPEAN PATENT APPLICATION

(11) **EP 3 933 846 A1**
(43) Date of publication of application: **05.01.2022**
(21) Application number: 19916823.8
(22) Date of filing: 15.08.2019
(51) Int. Cl.: G16H 30/20

(54) **THREE-DIMENSIONAL IMAGE MEASUREMENT METHOD, ELECTRONIC DEVICE, STORAGE MEDIUM, AND PROGRAM PRODUCT**

(30) Priority: 28.02.2019 CN 201910151898
(71) Applicant: VR Doctor Medical Technology (Shenzhen) Co., Ltd., Shenzhen, Guangdong, 518035 (CN)
(72) Inventor: LEE, David Wei, Shenzhen Guangdong 518035 (CN); LEE, Stewart Ping, Shenzhen Guangdong 518035 (CN)
(74) Representative: Dargiewicz, Joanna
(86) International application number: PCT/CN2019/100738
(87) International publication number: WO 2020/173052

(57) **Abstract**

An image three-dimensional measurement method, an electronic device, a storage medium and a program product are disclosed, wherein the image three-dimensional measurement method includes the following steps: performing feature extraction on a medical image scanned by a scanning device; performing image matching on the extracted medical image; performing calibration on the scanning device to determine an internal parameter of the scanning device; performing three-dimensional reconstruction on the medical image according to the internal parameter of the scanning device to obtain a three-dimensional image; and projecting the three-dimensional image onto a two-dimensional image, and calculating a size of a target area according to a mapping relationship between three-dimensional spatial points and two-dimensional image points. The method of the present disclosure realizes real-time measurement of the three-dimensional image by extracting the feature information of the image, image matching, calibration, reconstructing, measuring and the like, has the characteristics of high precision, good stability, non-contact measurement and the like, and is a practical measurement method with high automation degree.

## Description

### TECHNICAL FIELD

The disclosure relates to the field of medical imaging, and in particular to an image three-dimensional measurement method, an electronic device, a storage medium and a program product.

### BACKGROUND

The word "VRDS" refers to a head-mounted displays set for presenting virtual reality (VR). The three-dimensional stereo vision of VRDS medical images is a comprehensive science, involving mathematics, computer graphics, pattern recognition, digital image processing and digital signal processing. The vision research goal of VRDS medical image is to make VRDS system have the ability to recognize three-dimensional environmental information through two-dimensional images of medical Dicom volume data. Because the research results of medical image vision can be directly applied to Dicom (Digital Imaging and Communications in Medicine) volume data for measurement, volume data recognition, virtual reality and other operations in the medical field, the research on three-dimensional visual digital image processing of medical images has become the hottest topic in the world today.

In the prior art, although two-dimensional medical images can measure the size of target areas such as focus, for three-dimensional medical images, the accurate measurement function cannot be achieved.

Therefore, the prior art still needs to be improved and developed.

### SUMMARY

In view of the above shortcomings of the prior art, The present disclosure is intended to provide an image three-dimensional measurement method, an electronic device, a storage medium and a program product, aiming at solving the problem that the prior art cannot accurately measure three-dimensional medical images.

The technical solution of the present disclosure is as follows:
an image three-dimensional measurement method based on VRDS medical images, wherein, including the following steps:
performing a feature extraction on a medical image scanned by a scanning device;
performing an image matching on the extracted medical image;
performing a calibration on the scanning device to determine an internal parameter of the scanning device;
performing a three-dimensional reconstruction on the medical image according to the internal parameter of the scanning device to obtain a three-dimensional image;
and projecting the three-dimensional image onto a two-dimensional image, and calculating a size of a target area according to a mapping relationship between three-dimensional spatial points and two-dimensional image points.

The image three-dimensional measurement method based on VRDS medical image, wherein the three-dimensional image is projected onto the two-dimensional image by utilizing a pinhole camera model.

The image three-dimensional measurement method based on VRDS medical image, wherein after the step of projecting the three-dimensional image onto a two-dimensional image and calculating a size of a target area according to a mapping relationship between three-dimensional spatial points and two-dimensional image points, the method further includes:
scaling the three-dimensional image and keeping the calculated size when a scaling instruction for the three-dimensional image is received.

The image three-dimensional measurement method based on VRDS medical image, wherein the step of projecting the three-dimensional image onto a two-dimensional image and calculating a size of a target area according to a mapping relationship between three-dimensional spatial points and two-dimensional image points specifically includes:
projecting two measuring points in a target area onto a plane to obtain corresponding projection points;
calculating a measured length between the two projection points on the plane;
according to a proportion of the measured length to an actual length of the plane, obtaining an actual length between the two projection points; and calculating actual lengths between the measuring points and the corresponding projection point;
finally calculating a size of the target area.

The image three-dimensional measurement method based on VRDS medical image, wherein the feature extraction is a feature extraction method based on contour lines or based on image gray values.

The image three-dimensional measurement method based on VRDS medical image, wherein the image matching is an image matching method based on image gray information or based on image features.

The image three-dimensional measurement method based on VRDS medical image, wherein the calibration adopts a calibration method or a self-calibration method based on an active vision system.

An electronic device, wherein, includes:
a processor adapted to implement instructions, and
a storage device adapted to store a plurality of instructions adapted to be loaded and executed by the processor;
performing a feature extraction on a medical image scanned by a scanning device;
performing an image matching on the extracted medical image;
performing a calibration on the scanning device to determine an internal parameter of the scanning device;
performing a three-dimensional reconstruction on the medical image according to the internal parameter of the scanning device to obtain a three-dimensional image;
and projecting the three-dimensional image onto a two-dimensional image, and calculating a size of a target area according to a mapping relationship between three-dimensional spatial points and two-dimensional image points.

A non-transitory computer-readable storage medium, wherein the non-transitory computer readable storage medium stores a computer-executable instruction, and the computer readable instruction, when executed by one or more processors, can cause the one or more processors to execute the image three-dimensional measurement method based on VRDS medical images.

A computer program product, wherein the computer program product includes a computer program stored on a non-transitory computer-readable storage medium, the computer program includes program instructions, and the program instructions, when executed by a processor, cause the processor to execute the image three-dimensional measurement method based on VRDS medical images.

Advantageous effects: the method of the present disclosure realizes real-time measurement of the three-dimensional image by extracting the feature information of the image, image matching, calibration, reconstructing, measuring and the like, has the characteristics of high precision, good stability, non-contact measurement and the like, and is a practical measurement method with high automation degree.

### BRIEF DESCRIPTION OF THE DRAWINGS

Fig. 1 is a schematic flowchart of a preferred embodiment of an image three-dimensional measurement method of the present disclosure.
Fig. 2-4 are effect diagrams of the measurement method of the present disclosure in actual measurement.
Fig. 5 is a structural block diagram of an electronic device according to a preferred embodiment of the present disclosure.

### DETAILED DESCRIPTION OF THE EMBODIMENT

The present disclosure provides an image three-dimensional measurement method, an electronic device, a storage medium and a program product, in order to make the objectives, technical solutions and effect of the present disclosure clearer and explicit, the disclosure is further explained in detail below. It should be understood that the specific embodiments described herein are only for illustrating but not for limiting the present disclosure.

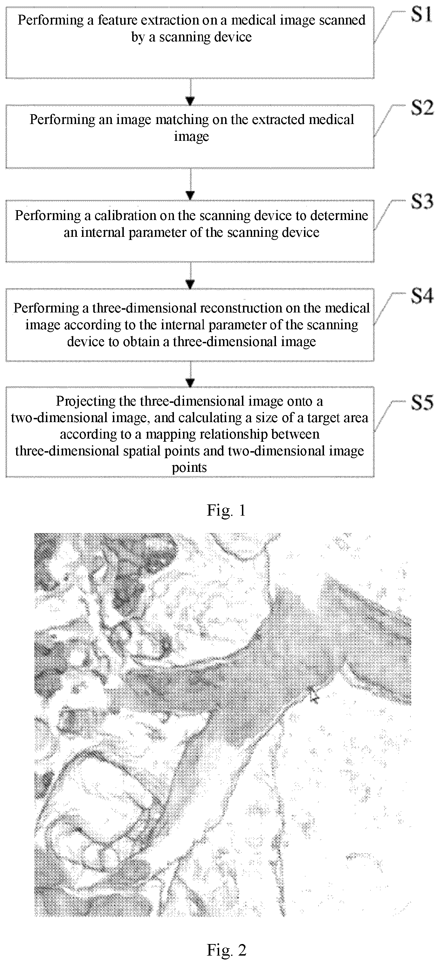

Referring to Fig. 1, Fig. 1 is a schematic flowchart of a preferred embodiment of an image three-dimensional measurement method based on VRDS medical images of the present disclosure, including the following steps:
S1, performing a feature extraction on a medical image scanned by a scanning device;
S2, performing an image matching on the extracted medical image;
S3, performing a calibration on the scanning device to determine an internal parameter of the scanning device;
S4, performing a three-dimensional reconstruction on the medical image according to the internal parameter of the scanning device to obtain a three-dimensional image;
S5, projecting the three-dimensional image onto a two-dimensional image, and calculating a size of a target area according to a mapping relationship between three-dimensional spatial points and two-dimensional image points.

It should be noted that the sequence numbers of the above steps are only for convenience, and do not represent the execution order of each step, according to different situations and requirements, the execution order of the steps can be slightly adjusted, which should belong to the protection scope of the present disclosure.

First, performing a feature extraction on a medical image scanned by a scanning device.

The scanned device is preferably a CT (computed tomography) /MRI (nuclear magnetic resonance) scanned device. It can also be DTI (diffusion tensor imaging) or PET-CT (positron emission computed tomography) scanned equipment.

In this step S1, the feature extraction is performed in order to obtain the image features on which matching depends, the image features obtained by feature extraction have distinguishability, invariance, stability and the ability to effectively solve ambiguous matching.

The image features used in the three-dimensional measurement method of VRDS 4D medical images have feature points, feature lines and feature areas. Wherein, the feature points usually refer to the points where the gray level changes drastically, including the intersection points of straight lines, the points where the curvature changes the most (corners or inflection points) on the contour of objects, and the solitary points on monotonous background, etc. Feature lines mainly refer to curves and edge segments of images, the edges in images reflect the discontinuity of object structure changes, which contains abundant information about object structure. The feature area is an area surrounded by feature lines.

Further, the feature extraction in the present disclosure is a feature extraction method based on image gray value or contour line.

Wherein, for the feature extraction method based on image gray value, the method first defines an operator, and extracts feature points by finding the extreme value of the operator on the gray image, this operator can not only detect edges, but also detect angular point, moreover, there are few parameters that need to be set artificially, and its accuracy and robustness are very good.

Wherein, for the feature extraction method based on contour line, first, the edge is extracted from the image, and then the maximum point of curvature is searched on the chain formed by the edge, or the edge is approximated by a polygon, and then the vertices of the polygon are calculated as feature points, and feature points are extracted from the edge contour line, in order to obtain robust results, a multi-scale framework is preferably introduced by the present disclosure. The contour curve is approximated by multi-splines function, and straight lines portion are extracted from the contour line where the curvature changes the most in B-spline function, these straight lines are grouped according to certain rules, and the intersection of straight lines in each group is the feature point.

Image matching is the most important problem in computer binocular vision. According to different matching features and methods, the image matching is an image matching method based on image features or image gray information.

For matching based on image feature, because the feature points of the image are much less than the pixel points, this matching method greatly reduces the calculation amount of the matching process; at the same time, the matching metric value of feature points is sensitive to the change of position, which can greatly improve the matching accuracy; moreover, the extraction process of feature points can reduce the influence of noise, and make matching have better adaptability to gray level change, image deformation and occlusion.

For matching based on image gray information, image gray information is generally divided into gray information and gray statistical information. According to the present disclosure, in the image matching algorithm of VRDS, correlation operation is mainly performed on the gray values in the spatial domain of two images, and the matching position is obtained according to the peak value of the correlation coefficient. The methods used can be normalized cross-correlation, statistical correlation, average absolute difference and average square difference; frequency domain correlation based on FFT frequency domain, including phase correlation and power spectrum correlation; and invariant moment matching, amplitude sorting correlation algorithm, FFT correlation algorithm and hierarchical search sequence judgment algorithm.

Calibration is an indispensable key step in the field of computer vision to acquire three-dimensional Euclidean information from two-dimensional images, because the Euclidean information of three-dimensional structure can not be obtained without calibration of CT/MRI scanned device, only projective reconstruction can be achieved, the calibration results of CT scanned device directly determine the quality of three-dimensional reconstruction results. There is a one-to-one correspondence relationship between the three-dimensional spatial points and the image points in their images, and their positional relationship is determined by the imaging geometric model of CT/MRI scanned device. Parameters of the geometric model are referred to as parameters in CT/MRI scanned device.

Further, the calibration adopts a calibration method or a self-calibration method based on an active vision system. Besides using of the above two calibration methods, traditional calibration methods can also be used.

For the traditional calibration method, it uses the corresponding constraint relationship between a standard reference object and its image to determine the internal parameters of CT/MRI scanned device, that is, an object with known shape and size is placed in front of the CT/MRI scanned device, which is called the calibration object, and the CT/MRI scanned device acquires the image of the calibration object, and then calculates the internal parameters of the camera (that is refer to the camera of CT/MRI scanned device, same as below). From the point of view of calculation, the traditional calibration methods of CT/MRI scanned device can be divided into four categories: calibration method using optimization algorithm, calibration method using transformation matrix of CT/MRI scanned device, two-step method of distortion compensation and biplane calibration method of imaging model of CT/MRI scanned device. The advantage of this traditional calibration method is that it can obtain higher accuracy.

For the calibration method based on active vision system, by controlling the motion of CT/MRI scanned device to obtain multiple images, the camera internal parameters are demarcated. Like the self-calibration method, the calibration method based on active vision system is a calibration method only using the corresponding relationship between images, and does not need high-precision calibration objects. Because some motion information of CT/MRI scanned device is obtained during the calibration process, the internal parameters of CT/MRI scanned device can be solved linearly, with simple calculation and good robustness.

As for the self-calibration method, it overcomes the shortcomings of the traditional method and the calibration method based on active vision system, it does not need calibration objects or strictly limit the movement of CT/MRI scanned device, and only relies on the geometric relationship between the corresponding points of multiple views to directly demarcate. In current, self-calibration methods mainly have self-calibration by solving the melting equation directly, hierarchical step-by-step calibration, self-calibration based on absolute quadric, modulus constraint calibration and hierarchical step-by-step calibration under variable internal parameters.

The self-calibration method is a method based on absolute conic or dual absolute quadric, the quasi-affine reconstruction is obtained from the constraint that all points on the image must be in front of CT/MRI scanned device, and then use this as initial value, the calibration is obtained by minimizing the difference between the internal parameters of CT/MRI scanned device that were attempted to be solved and those obtained by projective matrix decomposition of CT/MRI scanned device.

And performing three-dimensional reconstruction on the medical image according to the internal parameter of the scanning device to obtain a three-dimensional image. Wherein, three-dimensional reconstruction is to restore the three-dimensional information of objects.

Finally, after the three-dimensional structure of the scene is obtained from the image, it can be applied according to the actual needs. In the present disclosure, three-dimensional reconstruction and measurement of CT Dicom volume data can be performed, so the measurement method can also be called CT scanning measurement method.

The three-dimensional measurement system of VRDS medical image simulates human eye imaging geometry and projects three-dimensional scene onto two dimensional image. The three-dimensional measurement model of VRDS medical image describes the mapping relationship between 3D spatial points and 2D image points. The most specific and simple camera model is pinhole camera model, and projective geometry is the natural mathematical framework for describing this pinhole camera model. In projective spatial, both 3D spatial points and 2D image points can be represented by homogeneous coordinates, with the help of mathematical tools such as projective geometry and moment eyes, one can describe the imaging principle from three-dimensional spatial to two-dimensional image, the antipode geometry relationship between two images, and the calculation of reconstructing the shape of three-dimensional spatial objects from images.

First, several different types of coordinate systems are defined: world coordinate system, three-dimensional image coordinate system and VRDS 4D medical image camera coordinate system.

Wherein, world coordinate system: because the CT/MRI scanned device can be placed at any position in the hospital environment, the present disclosure selects the world coordinate system as the reference coordinate system to describe the scanned position of human body in the CT/MRI scanned device environment, which is called the world coordinate system, and the scale unit belongs to the physical unit.

Three-dimensional image coordinate system: The images collected by CT/MRI scanned device exist in the form of two-dimensional array, in order to measure the coordinate points after three-dimensional imaging, every element in the three-dimensional image with M rows and N columns is defined as a pixel, and its numerical value is the brightness of the three-dimensional image points. A three-dimensional rectangular coordinate system (u,v) is defined in the medical image of VRDS 4D, which is called the number of columns and rows of the three-dimensional image in the array. The three-dimensional image coordinate system represented by physical units (such as millimeters) created by VRDS 4D medical image, which takes a certain point within the three-dimensional image of human body as the basic origin, and the x-axis and y-axis will be parallel to the u-axis and v-axis respectively.

VRDS 4D medical image coordinate system: In order to acquire the geometric relationship between 2D and three-dimensional imaging after scanning by CT/MRI scanned device, a medical image coordinate system is defined. The x-axis and y-axis should be parallel to the x-axis and y-axis of three-dimensional image coordinate system respectively, z is the optical axis of CT/MRI scanned device and perpendicular to the central point plane of three-dimensional image, and the intersection of optical axis and three-dimensional image plane is the origin of VRDS 4D medical image coordinate system.

Further, in the three-dimensional measurement of VRDS 4D medical image, the pinhole camera model is used to project the three-dimensional image onto the two-dimensional image. The pinhole camera model is simple and practical without losing accuracy. In the three-dimensional measurement of VRDS 4D medical image, the point x with the spatial coordinate x=(x,y,z)t in the three-dimensional image is mapped to the point x in the two-dimensional image plane, which is the intersection point between the straight line connecting the point x and the projection center c and the image plane. The projection center of three-dimensional measurement of VRDS 4D medical image is called CT/MRI scanned device center, also called optical center. The vertical line from the center of CT/MRI scanned device to the image plane is called the main axis of CT/MRI scanned device, and the intersection point between the main axis and the two-dimensional image plane is called the main point.

Further, the steps of the projecting of the three-dimensional image onto a two-dimensional image, and calculating a size of a target area according to a mapping relationship between three-dimensional spatial points and two-dimensional image points further includes:
scaling the three-dimensional image and keeping the calculated size when a scaling instruction for the three-dimensional image is received.

In the three-dimensional measurement of VRDS 4D medical image, when the scale proportion is changed, the size of the measured target area does not change, and the size of the target area records the raw size of the object. That is to say, when performing three-dimensional measurement in real-time dynamic environment using VRDS 4D medical image, even if the operations such as scaling and moving are performed, the real size obtained by three-dimensional measurement will not change.

Further, the steps of the projecting of the three-dimensional image onto a two-dimensional image, and calculating a size of a target area according to a mapping relationship between three-dimensional spatial points and two-dimensional image points specifically includes:
projecting two measuring points in a target area onto a plane to obtain corresponding projection points;
calculating a measured length between the two projection points on the plane;
according to a proportion of the measured length to an actual length of the plane, obtaining an actual length between the two projection points; and calculating actual lengths between the measuring points and the corresponding projection point;
finally calculating a size of the target area.

The proportion of the above plane in actual length is also the actual physical scale of the plane.

For example, assume that two points A1 and A2 are points to be measured in a three-dimensional image
1. Project two points A1 and A2 to be measured in a three-dimensional image onto the plane of a two-dimensional image to obtain corresponding projection points V1 and V2, and calculate the measurement length between V1 and V2 through the coordinate (X1,Y1) of V1 in the plane and the coordinate (X2,Y2) of V2 in the plane of the two-dimensional image;
2. Calculate the proportion of the length between V1 and V2 and the plane in the actual length to obtain the actual length between V1 and V2;
3. Calculate the measured length between A1 and V1 and the measured length between A2 and V2;
4. The measured length between points A1 and A2 is obtained by pythagorean theorem of right triangle.

The effects of the final measurement are shown in Fig. 2 to 4. The three-dimensional measurement of VRDS 4D medical image of the present disclosure is mainly a measurement method which takes the image as a means of detecting and transmitting information, and finally acquires the actual information of the measured object from the image by extracting the feature information of the image. The three-dimensional measurement method of VRDS 4D medical image has strong adaptability in precision, speed, intelligence and other aspects, and has the characteristics of high precision, good stability, non-contact measurement and the like, and a practical measurement system with high automation degree is formed by combining image processing technology.

The VRDS 4D medical image of the present disclosure achieves three-dimensional measurement in 3D and 4D real-time dynamic environments. Also, the three-dimensional measurement of VRDS 4D medical image can test the abnormal phenomena of inner wall, inner diameter and outer wall of blood vessel. At the same time, the three-dimensional measurement of VRDS 4D medical image can be used for the three-dimensional measurement of the focus tumor in real-time dynamic environment. In addition, the three-dimensional measurement of VRDS 4D medical images can be used for the three-dimensional measurement of the focus organs and residual tissues after tumor resection in real-time dynamic environment.

In addition, the three-dimensional measurement of VRDS 4D medical images of the present disclosure can also be used for three-dimensional measurement of other targets in a real-time dynamic environment as required.

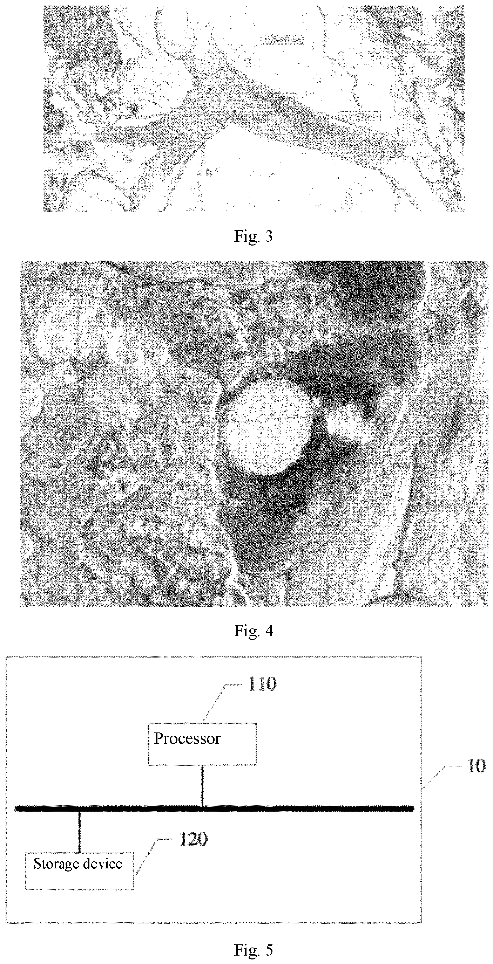

The present disclosure also provides an electronic device 10, as shown in Fig. 5, which includes:
a processor 110, adapted to implement instructions, and
a storage device 120, adapted to store a plurality of instructions adapted to be loaded and executed the following operation by the processor 110;
performing a feature extraction on a medical image scanned by a scanning device;
performing an image matching on the extracted medical image;
performing a calibration on the scanning device to determine an internal parameter of the scanning device;
performing a three-dimensional reconstruction on the medical image according to the internal parameter of the scanning device to obtain a three-dimensional image; and
projecting the three-dimensional image onto a two-dimensional image, and calculating a size of a target area according to a mapping relationship between three-dimensional spatial points and two-dimensional image points.

The processor 110 may be a general purpose processor, a digital signal processor (DSP), an application specific integrated circuit (ASIC), a field programmable gate array (FPGA), a single chip microcomputer, an ARM (Acorn RISC Machine) or other programmable logic devices, discrete gate or transistor logic, discrete hardware components or any combination of these components. Furthermore, the processor can also be any traditional processor, microprocessor or state machine. A processor may also be implemented as a combination of computing devices, for example, a combination of DSP and microprocessor, multiple microprocessors, one or more microprocessors in combination with DSP cores, any other such configuration.

As a non-volatile computer readable storage medium, the storage device 120 may be configured to store a non-volatile software program, a non-volatile computer executable program and a module, such as program instructions corresponding to the image three-dimensional measurement method based on VRDS medical images in the embodiment of the present disclosure. The processor executes various functional applications and data processing of the image three-dimensional measurement method based on VRDS medical images by running the nonvolatile software programs, instructions and units stored in the storage device, that is, achieves the above method embodiments.

The specific technical details of the above electronic device 10 have been detailed in the previous method, so here will not be described again for brevity.

The disclosure also provides a non-transitory computer-readable storage medium, the non-transitory computer-readable storage medium stores a computer-executable instruction, and the computer readable instruction, when executed by one or more processors, can cause the one or more processors to execute the image three-dimensional measurement method based on VRDS medical images.

The disclosure also provides a computer program product, the computer program product includes a computer program stored on a non-transitory computer-readable storage medium, the computer program includes program instructions, and the program instructions, when executed by a processor, cause the processor to execute the image three-dimensional measurement method based on VRDS medical images.

It should be understood that the application of the present disclosure is not limited to the above-described examples, and that those skilled in the art should recognize that modifications and variations are possible in the light of the above specifications, all such modifications and variations falling within the range of the appended claims.

## Claims

1. An image three-dimensional measurement method based on Virtual Reality Doctor system (VRDS) medical images, **characterized in that**,
performing a feature extraction on a medical image scanned by a scanning device;
performing an image matching on the extracted medical image;
performing a calibration on the scanning device to determine an internal parameter of the scanning device;
performing a three-dimensional reconstruction on the medical image according to the internal parameter of the scanning device to obtain a three-dimensional image;
and projecting the three-dimensional image onto a two-dimensional image, and calculating a size of a target area according to a mapping relationship between three-dimensional spatial points and two-dimensional image points.

2. The image three-dimensional measurement method based on VRDS medical image according to claim 1, **characterized in that** the three-dimensional image is projected onto the two-dimensional image by utilizing a pinhole camera model.

3. The image three-dimensional measurement method based on VRDS medical image according to claim 1, **characterized in that** after the step of projecting the three-dimensional image onto a two-dimensional image and calculating a size of a target area according to a mapping relationship between three-dimensional spatial points and two-dimensional image points, the method further comprises:
scaling the three-dimensional image and keeping the calculated size when a scaling instruction for the three-dimensional image is received.

4. The image three-dimensional measurement method based on VRDS medical image according to claim 1, **characterized in that** the step of projecting the three-dimensional image onto a two-dimensional image and calculating a size of a target area according to a mapping relationship between three-dimensional spatial points and two-dimensional image points specifically comprises:
projecting two measuring points in a target area onto a plane to obtain corresponding projection points;
calculating a measured length between the two projection points on the plane;
according to a proportion of the measured length to an actual length of the plane, obtaining an actual length between the two projection points; and calculating actual lengths between the measuring points and the corresponding projection point;
finally calculating a size of the target area.

5. The image three-dimensional measurement method based on VRDS medical image according to claim 1, **characterized in that** the feature extraction is a feature extraction method based on contour lines or based on image gray values.

6. The image three-dimensional measurement method based on VRDS medical image according to claim 1, **characterized in that** the image matching is an image matching method based on image gray information or based on image features.

7. The image three-dimensional measurement method based on VRDS medical image according to claim 1, **characterized in that** the calibration adopts a calibration method or a self-calibration method based on an active vision system.

8. An electronic device, **characterized in that**
a processor adapted to implement instructions, and
a storage device adapted to store a plurality of instructions adapted to be loaded and executed by the processor;
performing a feature extraction on a medical image scanned by a scanning device;
performing an image matching on the extracted medical image;
performing a calibration on the scanning device to determine an internal parameter of the scanning device;
performing a three-dimensional reconstruction on the medical image according to the internal parameter of the scanning device to obtain a three-dimensional image;
and projecting the three-dimensional image onto a two-dimensional image, and calculating a size of a target area according to a mapping relationship between three-dimensional spatial points and two-dimensional image points.

9. A non-transitory computer-readable storage medium, **characterized in that** the non-transitory computer readable storage medium stores a computer-executable instruction, and when the computer-executable instruction is executed by one or more processors, the one or more processors are to execute the image three-dimensional measurement method based on VRDS medical images according to any one of claims 1-7.

10. A computer program product, **characterized in that** the computer program product comprises a computer program stored on a non-transitory computer-readable storage medium, the computer program comprises program instructions, and when the program instructions are executed by a processor, the processor is to execute the image three-dimensional measurement method based on VRDS medical images according to any one of claims 1-7.
